# NEW EUROPEAN PATENT SPECIFICATION

(11) **EP 0 151 033 B2**
(45) Date of publication and mention of the opposition decision: **26.07.1995**
(45) Mention of the grant of the patent: 18.04.1990
(21) Application number: 85300626.0
(22) Date of filing: 30.01.1985
(51) Int. Cl.: A61F 13/15

(54) **Method of and improved apparatus for making discrete airlaid absorbent fibrous articles**
Verfahren und Gerät zur Erzeugung von diskreten, saugfähigen, aus durch Luftströmung angeführten Fasern bestehenden Artikeln
Procédé et appareil de fabrication d'articles discrets absorbants en fibre déposée par jet d'air

(30) Priority: 01.02.1984 US 576099
(43) Date of publication of application: 07.08.1985
(73) Proprietor: THE PROCTER & GAMBLE COMPANY, Cincinnati Ohio 45201 (US)
(72) Inventor: Peterson, David Albert, Cincinnati Ohio 45224 (US); Benson, Douglas Herrin, West Harrison Indiana (US)
(74) Representative: Bottema, Johan Jan

(56) References cited:
- DE-A- 3 203 992
- GB-A- 820 734
- SE-B- 325 003
- US-A- 2 282 477
- US-A- 3 518 726
- US-A- 3 846 871
- US-A- 4 005 957

## Description

### Technical field

This invention pertains to forming airlaid articles such as discrete absorbent cores for catamenial napkins or disposable diapers. More particulary it pertains to making such discrete articles so that they have high structural integrity, and good edge definition (i.e., the edge of the article being made in the image of the edge of the cavity). This also limits the degree of length/width growth of the articles if they are subjected to calendering after being removed from the cavities. This is, basically, achieved by compacting each airlaid mass prior to removing it from its formation cavity.

### Background art

A drum-type airlaying apparatus for making discrete absorbent fibrous articles is disclosed, for example, in U.S. Patent 2,073,329 which issued March 9, 1937 to C. P Winter, and which includes a broadly divergent duct which extends from his drylap disintegrator (i.e., means for air entraining fibers) to the periphery of his first deposition drum. Another drum-type airlaying apparatus for airlaying discrete absorbent fibrous articles which has a similarly broadly divergent duct for air-entrained fiber flow is disclosed in U.S. Patent 4,005,957 which issued February 1, 1977 to Peter P. Savich. A drum-type airlaying apparatus for making a concatinated stream of absorbent fibrous articles is disclosed in U.S. Patent 3,518,726 which issued July 7, 1970 to C.T. Banks. A belt-type airlaying apparatus having a duct for air-entrained fibers which is sharply angled obliquely downstream with respect to the laydown belt is disclosed in U.S. Patent 4,375,447 which issued March 1, 1983 to Raymond Chung. DE-A-3 203 992 discloses a method and apparatus for making sanitary napkins wherein the laid down fibre mass is subsequently subjected to compression.

### Disclosure of the invention

According to one aspect of the invention there is provided an airlaying apparatus for making discrete absorbent fibrous articles which apparatus includes a deposition chamber, a rotatable deposition drum having a plurality of article formation cavities therein disposed in circumferentially spaced relation about the periphery of said deposition drum, and wherein each of the cavities has side walls and a foraminous bottom wall secured to said side walls, means for directing air-entrained fibers towards a span of the drum passing through said deposition chamber, and means fo vacuum drawing the fiber-entrainment air through the foraminous bottom walls of the cavities and exhausting it from the apparatus, wherein the apparatus comprises
i) Scarfing means for removing fibers in excess of the quantity required to fill each said cavity to the level defined by the drum periphery; and
ii) means for compacting said articles by a predetermined amount prior to discharging each article from its respective formation cavity, said means comprising a lugged cylinder rotatable about an axis
disposed parallel to the axis of said rotatable drum in timed relation therewith, said cylinder having at least one lug which is sized and configured to precipitate said compaction by progressive engagement with the fibers in each said cavity in a rolling motion as said lugged cylinder and said deposition drum are rotated in timed relation. According to the method aspect of the present invention there is provided a method of making discrete absorbent fibrous articles by deposition of an air entrained suspension of fibers in a succession of cavities disposed about the periphery of a rotatable drum type deposition apparatus, each said cavity being formed with solid side walls and a foraminous bottom wall disposed radially inwardly of said periphery, comprising the steps of:
a) depositing air entrained fibers in a cavity so as to form a fibrous mass filling said cavity;
b) withdrawing at least part of the fiber entrainment air by vacuum through said foraminous bottom wall; and
c) discharging said mass of fibers from said cavity, characterised in that the method also comprises the steps of;
d) overfilling said cavity with said fibers;
e) continuously scarfing away the excess of said fibers to thereby form in each said cavity an uncompacted preform of a said article having the size and shape of said cavity and a radially outwardly facing surface defined by the periphery of said drum;
f) contacting the radially outwardly facing surface of said fibrous mass in said filled cavity with a lug adapted to enter into engagement with said cavity, said lug being supported on a cylinder adapted to rotate in timed relationship with said deposition drum, said lug compressing said fibrous mass radially inwardly in a progressive manner as said drum and said cylinder rotate; and
g) discharging said compressed fibrous mass from each said cavity in a radially outward direction.

### Brief description of the drawings

An embodiment of the apparatus aspect of the invention is illustrated in the accompanying drawings in which:
Figure 1 is a somewhat schematic, fragmentary side elevational view of an apparatus embodiment of the present invention.
Figure 2 is an enlarged scale, fragmentary perspective view of a deposition cavity disposed on the periphery of the deposition drum of the apparatus shown in Figure 1.
Figure 3 is an enlarged scale, fragmentary sectional view taken along section line 3-3 of Figure 1 which section line extends radially through the center of a deposition cavity disposed in the rim of the deposition drum of the apparatus shown in Figure 1.

### Detailed description of the invention

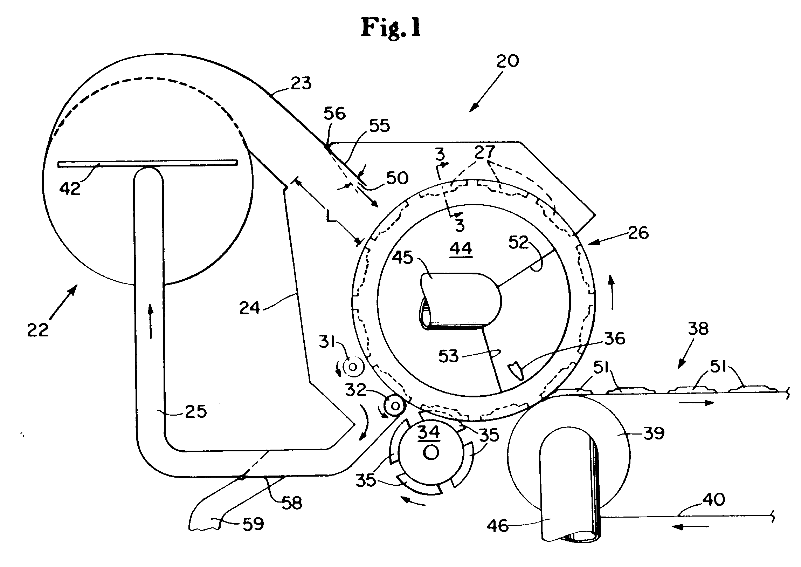

A fragmentary portion of an exemplary drum-type apparatus for making discrete absorbent fibrous articles such as cores of catamenial napkins or disposable diapers in accordance with the present invention is designated 20 in Figure 1. As further shown in Figure 1, apparatus 20 comprises a disc-type hammermill 22 having a columnar discharge chute 23, a hood 24, a recirculation manifold 25, a deposition drum 26 having a plurality of deposition cavities 27 disposed in circumferentially spaced relation about its periphery, two scarfing rolls 31 and 32, a lugged cylinder 34 having a plurality of radially extending lugs 35, a blow-off means or nozzle 36, and a take-away conveyor 38 comprising a vacuum-type return roll 39 and a foraminous endless belt 40. Means not shown are provided for feeding a drylap web into infeed slot 42 of hammermill 22 at a predetermined rate; means for powering and controlling hammermill 22; means for rotating drum 26, scarfing rolls 31 and 32, and conveyor 38 in time relation; means for maintaining a predetermined degree of vacuum in vacuum manifold 44 of drum 26 via vacuum duct 45, and means for maintaining a predetermined level of vacuum inside a sector of return roll 39 through vacuum duct 46. Additionally, a somewhat columnar stream 50 of air-entrained fibers is shown in Figure 1 to be exiting from the discharge chute 23 of hammermill 22 and directed generally radially towards a sector of drum 26 having a relatively small circumferential length; and an endless stream of discrete absorbent fibrous articles 51 is shown moving rightwardly on belt 40 of take-away conveyor 38. As used herein, a drylap web is a web of fibers which are subject to being disassociated and air-entrained by the action, for example, of hammermill 22.

Briefly, apparatus 20 comprises means for converting an endless length or roll of drylap web into a stream of discrete absorbent fibrous articles having good definition and structural integrity. The hammermill-alternately designated a fiberizer or disintegrator-disassociates the fibers of the drylap web and then discharges a relatively high velocity stream of air-entrained fibers, which stream is directed generally radially towards a relatively short circumferential span of the periphery of drum 26. The velocity- and mass-derived momentum of the fibers in the stream injects them into a deposition cavity disposed on the periphery of the drum while the substantially smaller momentum of the fiber-entrainment air enables the bulk of the fiber-entrainment air to turn upstream with respect to the periphery of the drum and be drawn through the foraminous bottom walls of substantially empty additional deposition cavities by the vacuum maintained in vacuum manifold 44 of drum 26. Indeed, as is more fully described hereinafter, each deposition cavity is preferably overfilled in its entirety (i.e., its full width and circumferential length), and the excess is scarfed away by scarfing rolls 31 and 32. Then, the mass of fibers disposed in each filled deposition cavity is compacted a predetermined amount by the action of a lug 35 on the lugged cylinder 34 to complete the formation of a discrete airlaid article 51 having good edge definition and structural integrity as a result of being compacted before being removed from its deposition cavity. The discrete articles are then transferred to the take-away conveyor by the joint action of the blow-off nozzle 36, and vacuum in a facing sector of the conveyor return roll 39. Parenthetically, the bulk of the fiber-entrainment air exits via substantially empty deposition cavities because of the flow impeding effect of the fiber build up in the deposition cavity passing under the stream 50 of air-entrained fibers.

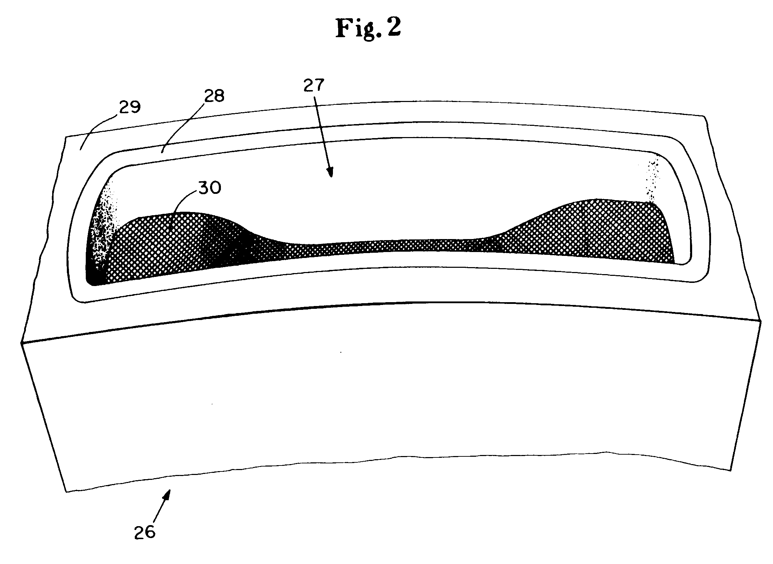

A deposition cavity 27 is shown in a perspective view, Figure 2, of a fragmentary portion of the periphery of deposition drum 26. The deposition cavity has a stepped configuration to provide articles 51 having substantially less basis weight (i.e., weight of fibers per unit of its plan-view area) in their end regions than their center spans. That is, as the articles 51 are formed they have generally uniform density, thick center spans and relatively thin end regions due to the geometry of the deposition cavities 27. Then, through the action of the lugged cylinder 34, the articles are compacted to achieve improved structural integrity; to have their edges more clearly defined; and to limit their length/width growth in the event they are further calendered or compacted after being removed from the deposition cavities. Additionally, the discrete articles 51 are preferably calendered and enveloped in suitable covering materials and such other converting operations as desired are effected downstream from the deposition member to produce finished disposable consumer products comprising airlaid fibrous cores; for example, catamenial napkins and/or disposable diapers.

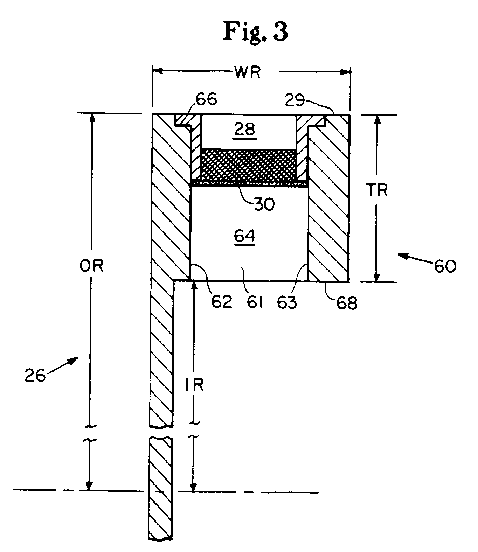

Referring now to Figure 3, a transverse-radial section of drum 26 is shown to have an outer radius OR and inner radius IR; a rim 60 having a radial thickness TR and a transverse width WR. The rim 60 as shown in Figure 3 has a radially extending hold 61 machined through it, which hole, as shown, is defined by side walls 62 and 63, and end walls 64, only one of the end walls being visible in the sectional view. Of course, hole 61 is only one of a plurality of such holes which are disposed in the rim of drum 26; one for each deposition cavity 27, Figure 1, which in the exemplary apparatus 20 number twelve which are spaced at thirty degrees center-to-center. The radially outwardly facing surface of the rim has a recess 66 machined in it to accommodate the flange of a deposition cavity insert 28 so that the radially outwardly facing surface of the insert is flush with the radially outwardly facing surface of the rim, and so that together they constitute the periphery 29 of the drum. A piece of screen or otherwise foraminous material is secured to the bottom end of insert 28 to constitute its bottom wall 30. The surface of the drum having a radius IR is designated sealing surface 68. The vacuum manifold 44, Figure 1, which is stationary (i.e., does not rotate with drum 26) is provided with sealing means which coacts with sealing surface 68, Figure 3, to enable the vacuum applied to the manifold to draw fiber-entrainment air downwardly through the cavities 27 and holes 61 which pass over the vacuum manifold as the drum rotates.

Referring again to Figure 1, apparatus 20 is shown to further comprises an optional baffle plate 55 which is pivotally mounted on the upstream lip of chute 23 by pivot pin 56; a recirculation dump valve 58; and a recirculation dump duct 59. The angular position of baffle plate 55 can be adjusted to precipitate a downstream velocity vector component to the stream 50 of fibers to match the peripheral velocity of drum 26, or to otherwise provide a sufficient downstream velocity vector component of stream 50 to achieve even filling of the deposition cavities 27. The recirculation dump valve 58, and the recirculation dump duct 59 are provided to divert airentrained fibers from the recirculation manifold 25 when apparatus 20 is turned off to prevent fibers in the recirculation manifold from precipitating deleterious ramifications during startups of apparatus 20.

### Exemplary embodiment of apparatus 20

An exemplary apparatus of the configuration shown in Figure 1 was sized and configured to make twelve articles 51 per revolution of the drum. In this apparatus, the articles are formed in deposition cavities having lengths of six-and-six-tenths inches (17.8 cm.) and which cavities are spaced eight-and-one-half inches (21.6 cm.) center-to-center about the periphery of the drum. The cavities are configured to be seven-tenths of an inch deep (1.8 cm) in their end regions, and one-and-three-tenths inches deep (3.3 cm) in their center spans in order to make articles 51 having nominal fiber weights of eight grams each. The hood wraps the drum approximately one-hundred-eighty degrees, centered about the upstream wall of the discharge chute 23 as indicated in Figure 1. The vacuum manifold 44 spans two-hundred-sixty degrees of the drum, and is so disposed that its upstream end 52 is subjacent the upstream end of the hood, and so that its downstream end 53 is positioned just upstream of the return roll 39 and nozzle 36. The lugged cylinder 34 is configured generally as shown with four lugs 35. The lugs are disposed on the same pitch as the pitch of the deposition cavities on drum 26 so that cylinder 34 and the drum mesh albeit preferably not in contacting relation) in a gear-like manner as they are rotated in synchronous relation. The disk hammermill 22 was obtained from Curt G. Joa. Inc., and is their hammermill model number 85R-9505-B. This hammermill acts somewhat like a quasi centrifugal air blower inasmuch as it draws air into its intake. Thus, by connecting the recirculation manifold 25 to the intake of the hammermill, no other means need be provided to effect flow in the recirculation manifold.

Apparatus 20 is preferably operated with the stream 50 having a length L of up to fourteen inches (35.6 cm.), and more preferably from ten to twelve inches (25.4 to 30.5 cm.); a velocity of stream 50 of from two-thousand to fifteen-thousand feet per minute (0.61 to 4.57 Km. per minute), and more preferably from six-thousand to ten-thousand feet per minute (1.83 to 3.05 Km. per minute); a flow rate of stream 50 of from one-thousand to fifteen-hundred cubic feet per minute (28.3 to 42.5 cubit meters per minute); a fiber to air weight ratio in stream 50 of from six-to-one to thirty-to-one, and more preferably from seven-to-one to sixteen-to-one; and a peripheral velocity of drum 26 preferably of from two-hundred-fifty to seven-hundred feet per minute (76.2 to 213 meters per minute).

The drylap used in the exemplary apparatus described above was obtained from The Buckeye Cellulose Corporation and comprised from seventy-five to one-hundred percent native softwood fibers, and from zero to twenty-five percent hardwood fibers.

## Claims

1. An airlaying apparatus (20) for making discrete absorbent fibrous articles (51) which apparatus includes a deposition chamber (24), a rotatable deposition drum (26) having a plurality of article formation cavities (27) therein disposed in circumferentially spaced relation about the periphery of said deposition drum (26) and wherein each of the cavities has side walls (62, 63, 64), and a foraminous bottom wall (30) secured to said side walls, means for directing air-entrained fibers towards a span of the drum (26) passing through said deposition chamber, and means for vacuum drawing the fiber-entrainment air through the foraminous bottom walls (30) of the cavities and exhausting it from the apparatus, characterised in that the apparatus comprises
i) Scarfing means (31, 32) for removing fibers in excess of the quantity required to fill each said cavity to the level defined by the drum periphery; and
ii) means for compacting said articles by a predetermined amount prior to discharging each article from its respective formation cavity, said means comprising a lugged cylinder (34) rotatable about an axis disposed parallel to the axis of said rotatable drum in timed relation therewith, said cylinder having at least one lug (35) which is sized and configured to precipitate said compaction by progressive engagement with the fibers in each said cavity (27) in a rolling motion as said lugged cylinder (34) and said deposition drum (26) are rotated in timed relation.

2. A method of making discrete absorbent fibrous articles by deposition of an air entrained suspension of fibers in a succession of cavities disposed about the periphery of a rotatable drum type deposition apparatus, each said cavity being formed with solid side walls and a foraminous bottom wall disposed radially inwardly of said periphery comprising the steps of;
a) depositing air entrained fibers in a cavity so as to form a fibrous mass filling said cavity;
b) withdrawing at least part of the fiber entrainment air by vacuum through said foraminous bottom wall; and
c) discharging said mass of fibers from said cavity, characterised in that the method also comprises the steps of;
d) overfilling said cavity with said fibers;
e) continuously scarfing away the excess of said fibers to thereby form in each said cavity an uncompacted preform of a said article having the size and shape of said cavity and a radially outwardly facing surface defined by the periphery of said drum;
f) contacting the radially outwardly facing surface of said fibrous mass in said filled cavity with a lug adapted to enter into engagement with said cavity, said lug being supported on a cylinder adapted to rotate in timed relationship with said deposition drum, said lug compressing said fibrous mass radially inwardly in a progressive manner as said drum and said cylinder rotate; and
g) discharging said compressed fibrous mass from each said cavity in a radially outward direction.

## Patentansprüche

1. Eine luftschichtende Vorrichtung (20) zur Herstellung von aus diskreten, saugfähigen Fasern bestehender Artikel (51), wobei die Vorrichtung umfaßt eine Ablagekammer (24) und eine rotierbare Ablagetrommel (26) mit einer Vielzahl von Hohlräumen (27) zur Gestaltung der Artikel, welche entlang des Umfangs dieser Ablagetrommel (26) angeordnet sind, und wobei jeder der Hohlräume Seitenwände (62, 63, 64) und einen gelöcherten Boden (30) aufweist, welcher an diesen Seitenwänden befestigt ist, Mittel, um die mit Luft eingetragenen Fasern zu einem Bereich der Trommel (26) hinzuleiten, der diese Ablagekammer passiert, und Mittel, um Luft zum Eintragen der Fasern durch die gelöcherten Böden (30) der Hohlräume abzusaugen und aus der Vorrichtung auszupumpen,
dadurch gekennzeichnet, daß die Vorrichtung umfaßt:
(i) Abschröpfmittel (31, 32) zum Entfernen überschüssiger Fasern auf eine Menge, die erforderlich ist, um jeden Hohlraum auf das durch den Umfang der Trommel definierte Maß zu füllen; und
(ii) Mittel zum Verdichten dieser Artikel in vorgegebenem Umfang vor der Entladung eines jeden Artikels aus seinem entsprechenden Gestaltungshohlraum, wobei diese Mittel einen Zylinder mit Vorsprüngen (34) umfassen, welcher um eine parallel zur Achse der rotierbaren Trommel angeordnete Achse hat und in zeitlicher Relation zu dieser rotieren kann, wobei dieser Zylinder wenigstens einen Vorsprung (35) hat, welcher so bemessen und angeordnet ist, daß diese Verdichtung durch fortschreitenden Kontakt mit den Fasern in jedem Hohlraum (27) während der rollenden Bewegung herbeigeführt wird, wenn der Zylinder mit Vorsprüngen (34) und die Ablagetrommel (26) zeitlich abgestimmt sich drehen.

2. Verfahren zur Herstellung von aus diskreten, saugfähigen Fasern bestehender Artikel durch Ablegung einer mit Luft eingetragenen Suspension von Fasern in eine Folge von Hohlräumen, die um den Umfang einer rotierbaren trommelartigen Ablagevorrichtung herum angeordnet sind, wobei jeder Hohlraum mit festen Seitenwänden und einem durchlöcherten Boden gebildet wird, die radial ins Innere dieses Umfangs angeordnet sind, umfassend die Schritte:
a) Ablegen der mit Luft eingetragenen Fasern in einen Hohlraum, so daß eine faserige Masse, die den Hohlraum ausfüllt, gebildet wird,
b) Absaugen wenigstens eines Teils der Fasereintragungsluft durch ein Vakuum durch den gelöcherten Boden und
c) Entladen dieser faserigen Masse aus dem Hohlraum,
dadurch gekennzeichnet, daß das Verfahren weiterhin die Schritte umfaßt:
d) jeden Hohlraum mit diesen Fasern überfüllen;
e) kontinuierlich die überschüssigen Fasern abschröpfen, um dabei in jedem Hohlraum eine nicht verdichtete Vorform dieses Artikels zu bilden, der die Größe und Form dieses Hohlraums aufweist und eine radial nach außen gerichtete Oberfläche aufweist, welche durch den Umfang der Trommel definiert ist;
f) Inberührungbringen der radial nach außen gerichteten Oberfläche dieser faserigen Masse in dem gefüllten Hohlraum mit einem Vorsprung, der so angeordnet ist, daß er mit dem Hohlraum in Verbindung kommt, wobei dieser Vorsprung auf einem Zylinder angebracht ist, der in zeitlich festgelegter Relation zur Ablagetrommel rotiert, und der Vorsprung die Fasermasse radial nach innen fortschreitend zusammenpreßt, während die Trommel und der Zylinder rotieren, und
g) Entladen dieser zusammengepreßten faserigen Masse aus jedem Hohlraum in radial auswärtiger Richtung.

## Revendications

1. Dispositif d'application à jet d'air (20) servant à fabriquer des articles fibreux absorbants discrets (51), ce dispositif incluant une chambre de dépôt (24), un tambour rotatif de dépôt (26) comportant une pluralité de cavités (27) de formation des articles, espacées circonférentiellement sur la périphérie dudit tambour de dépôt (26), et dans lequel chacune des cavités possède des parois latérales (62, 63, 64) et une paroi de fond perforée (30) fixée auxdites parois latérales, des moyens pour diriger des fibres entraînées par l'air en direction d'une zone du tambour (26) passant devant ladite chambre de dépôt, et des moyens pour entraîner par dépression l'air d'entraînement des fibres, à travers les parois de fond perforées (30) des cavités et l'évacuer hors du dispositif, caractérisé en ce que le dispositif comprend :
(i) des moyens d'évacuation (31, 32) pour éliminer les fibres en excès de la quantité nécessaire pour remplir chaque cavité au niveau défini par la périphérie du tambour ; et
(ii) des moyens pour tasser lesdits articles à un degré prédéterminé avant d'évacuer chaque article de sa cavité respective de formation, lesdits moyens comprenant un cylindre (34) comportant des parties saillantes et pouvant tourner autour d'un axe parallèle à l'axe dudit tambour rotatif, d'une manière synchronisée avec ce dernier, ledit cylindre possédant au moins une partie saillante (35) dimensionnée et conformée de manière à intensifier ledit tassement par suite de sa venue progressive en contact avec les fibres situées dans chacune desdites cavités (27) au cours d'un mouvement de roulement lorsque ledit cylindre (34) muni de parties saillantes et ledit tambour de dépôt (26) tournent d'une manière synchronisée.

2. Procédé pour fabriquer des articles fibreux absorbants discrets grâce au dépôt d'une suspension de fibres, entraînée par l'air, dans une succession de cavités disposées sur la périphérie d'un dispositif de dépôt du type à tambour rotatif, chacune desdites cavités étant formée avec des parois latérales pleines et une paroi de fond perforée, disposées intérieurement, du point de vue radial, par rapport à ladite périphérie, et incluant les étapes consistant à :
a) déposer des fibres entraînées par l'air dans une cavité de manière à former une masse fibreuse remplissant ladite cavité ;
b) retirer au moins une partie de l'air d'entraînement des fibres par dépression à travers ladite paroi de fond perforée ; et
c) refouler ladite masse de fibres comprimées hors de ladite cavité ;
caractérisée en ce que le procédé inclut également les étapes consistant à :
d) remplir à plein ladite cavité avec lesdites fibres ;
e) évacuer en continu l'excès desdites fibres afin de former dans chacune desdites cavités une préforme non tassée d'un article ayant la dimension et la forme de ladite cavité et une surface tournée radialement vers l'extérieur définie par la périphérie dudit tambour ;
f) amener en contact avec la surface, tournée radialement vers l'extérieur, de ladite masse fibreuse située dans ladite cavité remplie, une partie saillante apte à s'engager dans ladite cavité, ladite partie saillante étant supportée par un cylindre apte à tourner d'une manière synchronisée avec ledit tambour de dépôt, ladite partie saillante comprimant radialement vers l'intérieur ladite masse fibreuse d'une manière progressive lorsque ledit tambour et ledit cylindre tournent ; et
g) évacuer ladite masse fibreuse comprimée hors de chacune desdites cavités radialement vers l'extérieur.
